(19) Europäisches Patentamt
European Patent Office
Office européen des brevets

(11) Publication number: **0 231 089 B1**

# (12) EUROPEAN PATENT SPECIFICATION

(45) Date of publication of patent specification: **30.09.92**

(51) Int. Cl.5: **C12P 41/00**, C12P 7/62, C12P 7/64

(21) Application number: **87300437.8**

(22) Date of filing: **19.01.87**

## (54) Process for producing an optically active alcohol by a biochemical method.

(30) Priority: **21.01.86 JP 8997/86**

(43) Date of publication of application:
**05.08.87 Bulletin 87/32**

(45) Publication of the grant of the patent:
**30.09.92 Bulletin 92/40**

(84) Designated Contracting States:
**DE FR GB**

(56) References cited:
**EP-A- 0 064 855**
**EP-A- 0 126 416**

**J. AM. CHEM. SOC., vol. 107, 1985, pages 7072-7076, American Chemical Society; G. KIRCHNER et al.: "Resolution of racemic mixtures via lipase catalysis in organic solvents"**

**J. AM. CHEM. SOC., vol. 106, 1984, pages 2687-2692, American Chemical Society; B. CAMBOU et al.: "Preparative production of optically active esters and alcohols using esterase-catalyzed stereospecific transesterification in organic media"**

**PROC. NATL. ACAD. SCI. USA, vol. 82, May 1985, pages 3192-3196; A. ZAKS et al.: "Enzyme-catalyzed processed in organic solvents"**

(73) Proprietor: **Chisso Corporation**
**6-32, Nakanoshima 3-chome Kita-ku**
**Osaka-shi Osaka-fu(JP)**

(72) Inventor: **Yoshida, Naoyuki**
**2-A7-202 Kajiwara 5-chome Kamakura-shi**
**Kanagawa-ken(JP)**
Inventor: **Morita, Hiroshi**
**10-1-401 Seto Kanazawa-ku**
**Yokohama-shi, Kanagawa-ken(JP)**

(74) Representative: **Whalley, Kevin et al**
**MARKS & CLERK 57/60 Lincoln's Inn Fields**
**London WC2A 3LS(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 231 089 B1

## Description

This invention relates to a process for producing optically active alcohols by a biochemical method in which secondary alcohols are reacted with triglycerides in the presence of enzymes.

Optically active alcohols are known as chemical compounds which have a great demand for physiologically active substances, such as medical supplies, agricultural chemicals and so on, or these intermediates.

However, secondary alcohols represented by the formula:

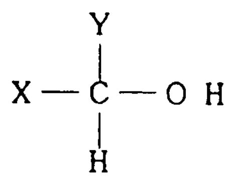

wherein X indicates an alkyl group having a carbon number of 2-10, Y indicates an alkyl group having a carbon number of 1-3, $CF_3$ or CN, and X ≠ Y, have optical isomers, so that these alcohols do not sufficiently exhibit activity in many cases unless either R- or S-alcohol is purely contained .

For the above reason, in order to obtain optically active substances, it needs to optically resolve racemates which are obtained by a common method of synthetic chemical preparation, to conduct asymmetric synthesis or to synthesize from optically active materials by a stereochemical method.

Accordingly, it is desired to develop a technique for optically resolving of the secondary alcohols by an industrially advantageous method.

The known optical resolution method of the secondary alcohols also needs complex steps and expensive optically active substance. For example, the salt is prepared by the steps in which 2-butanol or 2-octanol is reacted with phthalic anhydride, the obtained alkyl hydrogen phthalate is reacted with brucine, and the corresponding salt is repeatedly crystalized to resolve 2-butanol or 2-octanol. (Ref. Org. Syntheses, Coll. Vol. I, 418, 2nd ed., 1941).

Further, it is known that the optically active substances are obtained by optical resolution of biochemical methods.

For example, a method of Klibanov et al. (J. Am. Chem. Soc., 106, 2687 (1984)) uses yeast lipase or pig liber carboxy esterase dissolved in an aqueous buffered solution. In this case, unnecessary hydrolysis of triglyceride is unavoidable in the presence of moisture. Moreover, enzyme is soluble in water and unstable to moisture. In order to use the enzyme in a stable condition, it must be immobilized on a polymer. In other words, it is unable to remove or reuse the enzyme unless it is immobilized on the polymer.

There are biochemical methods in addition to the above method (Japanese Publication of Unexamined Patent Application No. Sho 59-205989 and others). In any case, it needs to use a buffered solution or lower alcohol, and an enzyme must be immobilized on a carrier.

Klibanov et al. also reported a method in which enzyme powder was directly added to a reaction system (J. Am. Chem. Soc., 107, 7072 (1985)). In this case, heptane or ether is used as its solvent, and not triglyceride but monoester is used as an ester.

This inventors had a research for resolving the above problems and obtaining a process for producing an optically active secondary alcohol by an advantageous industrial method and then found that a racemic secondary alcohol of a raw material is efficiently resolved to an optically active aliphatic ester and its antipode, namely an optically active alcohol, by a biochemically transesterification reaction.

An object of the invention to provide a process for producing an optically active alcohol by a biochemical method, in which the alcohol is efficiently obtained by an industrially advantageous method.

Namely, this invention provides a process for producing an optically active alcohol that comprises using an enzyme having the ability to conduct preferentially a transesterification reaction with a triglyceride and an (R,S)-alcohol represented by a general formula:

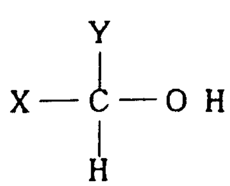

(1)

wherein X indicates alkyl group having a carbon number of 2-10, Y indicates alkyl group having a carbon number of 1-3, $CF_3$ or CN, and X ≠ Y, reacting the (R, S)-alcohol of the above formula (1) and the triglyceride to conduct the transesterification reaction in a solvent-free system under substantially anhydrous conditions and resolving the resulting ester to obtain an optically active alcohol which contains richly either R- or S-alcohol. The above reaction is represented by the following chemical equation:

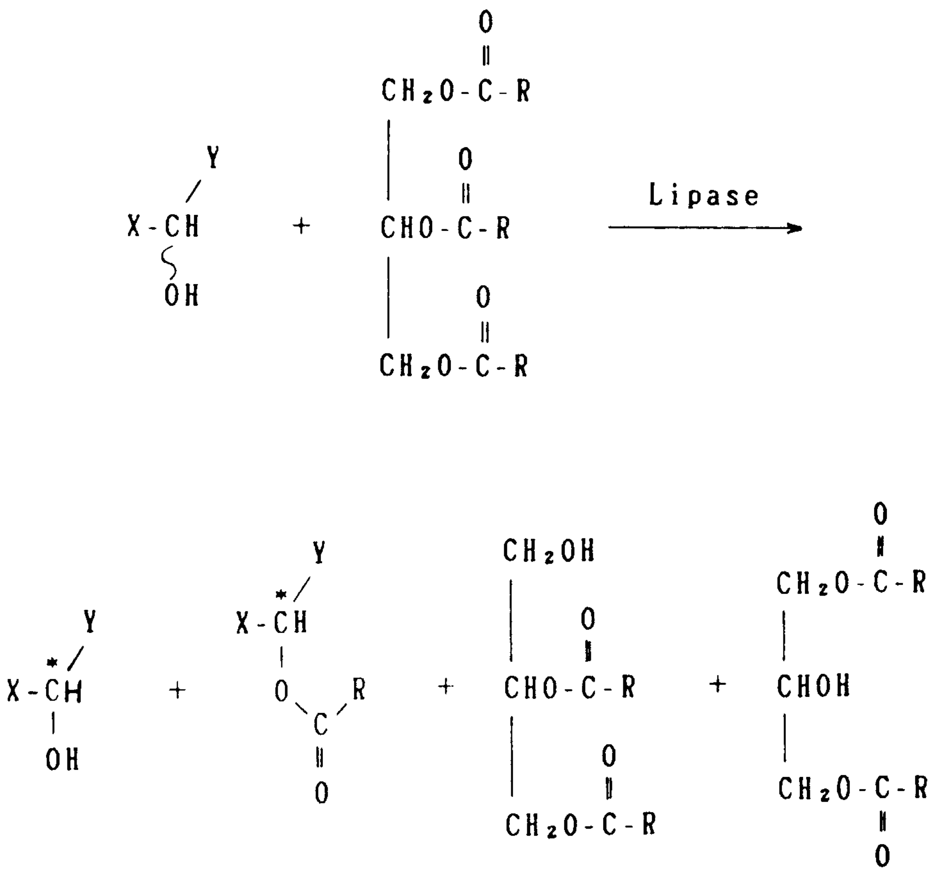


S- and R-

or

R- and S-

According to the method of this invention in comparison with that of the above Klibanov et al., the triglyceride does not hydrolyze because the method does not need the use of a small amount of water or alcohol instead of water, so that the enzyme is stably kept in organic solvent and easily separated after the reaction and its reuse. Furthermore, as the method of this invention is kept from free contaminant of micro-organism, there is no necessity for preparing special equipments, antiseptics, etc. It is possible to conduct the reaction in an open system.

The following description illustrates this invention more specifically.

In this invention, the (R, S)-alcohols of the raw materials are compounds which are easily available and can be synthesized without difficulty. It is enough to use the compounds which are commercially available such as 2-butanol, 2-pentanol, 2-hexanol, 2-heptanol, 2-octanol, 2-nonanol, 2-decanol etc.. Moreover, a compound which is $Y = CF_3$ in the formula (1), can be synthesized by the method of Campbell et al. (J. Am. Chem. Soc., 72, 4380 (1950)). A compound which is Y = CN in the formula (1), is easily also obtainable by the reaction of an aldehyde with hydrogen cyanide.

It is also enough to use the triglyceride which is commercially available without any difficulty, such as triacetin, tripropyonin, tributyrin, tristearin, trilaurin, trimyristin, triolein etc..

As the enzyme which is used in this invention, a lipase of pseudomonas genus is preferable in paticular. If the enzyme has ability of transesterification reaction preferentially between either the R- or S-alcohol and triglyceride when the enzyme is reacted with the (R, S)-alcohol, the enzyme can be used regardless its class. The following table shows commercially available enzyme that can be used in this reaction.

| Trade name | Origin | Seller or Maker |
|---|---|---|
| Lipase AP | Aspergillus niger | Amano Pharmaceutical Co.,Ltd |
| Lipase M | Mucor javanicus | 〃 |
| Lipase P | Pseudomonas fluorescens | 〃 |
| Lipase CES | Pseudomonas sp | 〃 |
| Lipase CE | Humicola lanuginosa | 〃 |
| Lipase F-AP | Rhizopus javanicus | 〃 |
| Lipase II | Porcine Pancreas | Sigma Chemical Co. |
| Lipase VII | Geotrichum Candidum | 〃 |
| Lipase X | Rhizopus delamar | 〃 |
| Lipase | Chromobacterium viscosum | Toyo Jozo Co., Ltd. |
| Palatase A | Aspergillus niger | Novo Industi A/S |
| Lipase | Rhiaopus niveus | Nagase Biochemicals, Ltd. |

Micro-organisms which produce the enzymes having the above ability can be used regardless their species and genus. These micro-organisms are Arthrobactor genus, Acromobacter genus, Chromobacterium genus, Candida genus, Mucor genus, Pseudomonas genus, Phizopus genus etc..

In practice of the invention, (R, S)-alcohols and triglycerides can be used without any particular treatments.

The transesterification reaction of an (R, S)-alcohol is conducted by mixing the (R, S)-alcohol with a triglyceride and contacting efficiently the mixture with an enzyme. Its reaction temperature is suitably 20-70 °C and preferably 30-45 °C. Its reaction time is widely 48-1000 hours. Short reaction time depends on elevated reaction temperature, raised activity of the enzyme and lowered concentration of substrates.

The (R, S)-alcohol and the triglyceride which are substrates, are mixed in the ratio 1:0.5 - 1:5 by mole and preferably 1:1.2 - 1:2.

After the transesterification reaction, the enzyme can be removed by common filter operation and used again as it is. The filtrate can be separated into an optically active alcohol and an ester. The obtained ester is commonly hydrolyzed in an alkali solution to derive an optically active alcohol which is an antipode of the above alcohol.

By the above described process, an optically R- and S-alcohol can be obtained.

The effects of this invention are as follows.

(1) Triglyceride is hardly hydrolyzed because it is substantially reacted under the conditions of water free.

(2) The enzyme can be easily recovered and reused.

(3) No special equipments and materials are used because the reaction can be conducted under the conditions of relatively lower temperatures and an open system.

(4) Optically active substances having high purity are obtained by one step reaction.

The following Examples illustrate this invention more specifically, but these will not always be precise in practical applications.

Example 1

10g of enzyme (produced by Amano pharmaceutical Co. Ltd., lipase "Amano" P), 65.1g (0.05mol) of (R,S)-2-octanol and 208.0g (0.69mol) of tributyrin were charged into three-necked flask and reacted with stirring for 36 days at 35 °C. After the reaction, (at this point, by detecting the ratio of compounds in the reaction solution with GC analysis, the ratio was 14.9% of 2-octanol, 27.0% of 2-octylbutylate, 17.8% of dibutyrin and 36.1% of tributyrin) the enzyme was removed by filtration and washed in toluene. The solution was added to the filtrate and then distilled under vacuum.

22.8g of S-(+)-2-octanol (yield : 70%, >99%ee) was obtained at the boiling point of 78-82 °C/14mmHg (1866 Pa), and 42.9g of R-(-)-2-octyl butylate (yield : 73%. 72%ee) was obtained at the boiling point of 112°C/14mmHg, respectively.

Finally, R-(-)-2-octyl butylate was hydrolyzed in alkali solution to obtain R-(-)-2-octanol (yield : 98%, 72%ee).

The obtained compounds were identified by structure analysis with NMR and optical activity with a polarimeter. Furthermore, the optical purity was determined by the comparison of the specific rotation between the obtained compounds and authentic samples.

Comparison Example 1

Using the same reactor as used in Example 1, the enzyme, 2-octanol and tributyrin were reacted under the same conditions as in Example 1 except that 50ml of 0.1M KPB (pH 8.0) was added. After 77 hours, the compounds in the reaction solution were detected with GC analysis, the ratio was 14.5% of butylic acid, 46.4% of 2-octanol, 4.0% of 2-octyl butylate, 16.1% of dibutyrin and 15.5% of tributyrin.

The result shows that the hydrolysis of tributyrin is more preferential than the transesterification reaction in the presence of a small amount of water, so that the optical resolution could not be conducted.

Example 2

Using the same enzyme as used in Example 1 under the same conditions as in Example 1, 23.4g of S-(+)-2-octanol (yield : 71%, >99%ee) and 46.2g of R-(-)-2-octyl butylate (yield : 92%, 69%ee) were obtained.

Moreover, the above enzyme was used under the same conditions as in Example 1 several times, and its deactivation was hardly found during the reaction.

Then the aimed S-(+)-2-octanol and R-(-)-2-octyl butylate were obtained.

The obtained compounds were identified and determined by the same method as in Example 1.

Example 3

40g of the same enzyme as used in Example 1, 158. 3g (1 mol) of (R,S)-2-decanol and 332.6g (1.1 mol) of tributyrin were reacted under the same conditions as in Example 1, 64g of S-(+)-2-decanol (yield : 81%, >78%ee) was obtained at the boiling point of 84°C/4.5mmHg, and 105g of R-(-)-2-octyl butylate (yield : 92%, 71%ee) was obtained at the boiling point of 107°C/3.5mHg, respectively.

The obtained compounds were identified and determined by the same method as in Example 1.

Example 4

Using the same reactor under the same conditions as in Example 1 except that (R,S)-2-octanol was replaced by (R,S)-2-pentanol. S-(+)-2-pentanol (yield : 73.2%, >62%ee) and R-(-)-2-pentyl butylate (yield : 82.2%) were obtained, respectively.

The obtained compounds were identified and determined by the same method as in Example 1.

Example 5

Using the same reactor under the same conditions as in Example 1, except that tributyrin was replaced by tripropionin, S-(+)-2-octanol (yield : 71%, >91%ee) and R-(-)-2-octyl propionate (yield : 86%) were

obtained, respectively.

The obtained compounds were identified and determined by the same method as in Example 1.

Example 6 ~ 8

Using the same method under the same conditions as in Example 1, optically active secondary alcohols having yields as shown in the following table were obtained.

| Example | Alcohol | S | | R | |
|---|---|---|---|---|---|
| | | yield(%) | ee(%) | yield(%) | ee(%) |
| 6 | 2-hexanol | 68 | 93 | 67 | 73 |
| 7 | 2-heptanol | 76 | 86 | 69 | 74 |
| 8 | 2-nonanol | 61 | 89 | 80 | 52 |
| 9 | 3-nonanol | 60 | 88 | 93 | 75 |

This invention is applied to a biochemical process for producing optically active alcohols known as chemical compounds which have a great demand for physiologically active substances, such as medical supplies, agricultural chemicals and so on, or these intermediates.

## Claims

1. A process for producing an optically active alcohol, characterized by using an enzyme having the ability to conduct preferentially a transesterification reaction with a triglyceride and an (R,S)-alcohol represented by a general formula:

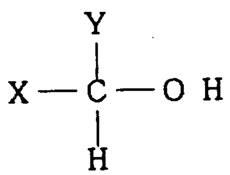

(1)

wherein X indicates an alkyl group having 2-10 carbon atoms, Y indicates an alkyl group having 1-3 carbon atoms, $CF_3$ or CN, and X is not the same as Y, reacting the (R,S)-alcohol of the above formula (1) and the triglyceride in a solvent-free system to conduct the transferication reaction under substantially anhydrous conditions and resolving the resulting ester to obtain an optically active alcohol which contains richly either R- or S-alcohol.

2. A process as claimed in claim 1, characterized in that the enzyme is a lipase of Pseudomonas genus .

3. A process as claimed in claim 1 or 2, characterized in that Y of the formula (1) is an alkyl group having 1 - 3 carbon atoms.

4. A process as claimed in claim 1 or 2, characterized in that Y of the formula (1) is $CH_3$ or $C_2H_5$.

5. A process as claimed in claim 2, characterized in that Y of the formula (1) is $CF_3$.

## Patentansprüche

1. Ein Verfahren zur Herstellung eines optisch aktiven Alkohols, gekennzeichnet durch die Verwendung eines Enzyms mit der Fähigkeit, vorzugsweise eine Umesterungsreaktion mit einem Triglyzerinester und einem (R,S)-Alkohol, dargestellt durch die allgemeine Formel:

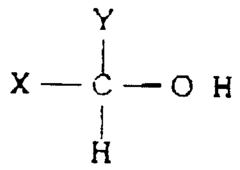

(1)

durchzuführen, bei der X eine Alkylgruppe mit 2 bis 10 Kohlenstoffatomen bedeutet, Y eine Alkylgruppe mit 1-3 Kohlenstoffatomen, $CF_3$ oder CN bedeutet, und X nicht das gleiche wie Y ist, durch das Eingehen einer Reaktion des (R,S)-Alkohols der obigen Formel (1) und des Triglyzerinesters in einem lösungsmittelfreien System, um die Umesterungsreaktion im wesentlichen unter wasserfreien Bedingungen durchzuführen, und durch das Zerlegen des resultierenden Esters, um einen optisch aktiven Alkohol zu erhalten, der reichlich entweder R- oder S-Alkohol enthält.

2. Ein Verfahren nach Anspruch 1, dadurch gekennzeichnet, daß das Enzym eine Lipase von Pseudomonas genus ist.

3. Ein Verfahren nach Anspruch 1 oder 2, dadurch gekennzeichnet, daß Y der Formel (1) eine Alkylgruppe mit 1-3 Kohlenstoffatomen ist.

4. Ein Verfahren nach Anspruch 1 oder 2, dadurch gekennzeichnet, daß Y der Formel (1) $CH_3$ oder $C_2H_5$ ist.

5. Ein Verfahren nach Anspruch 2, dadurch gekennzeichnet, daß Y der Formel (1) $CF_3$ ist.

**Revendications**

1. Un procédé de production d'un alcool optiquement actif, caractérisé par le fait qu'on utilise une enzyme ayant la capacité d'effectuer de préférence une réaction de transestérification avec un triglycéride et un (R,S)-alcool représenté par une formule générale:

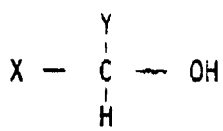

(1)

dans laquelle X indique un groupement alkyle ayant de 2 à 10 atomes de carbone, Y indique un groupement alkyle ayant de 1 à 3 atomes de carbone, $CF_3$ ou CN, et X n'est pas le même que Y, qu'on fait réagir le (R,S)-alcool de la formule (1) ci-dessus et le triglycéride dans un système sans solvant pour effectuer la réaction de transestérification dans des conditions substantiellement anhydre et qu'on procède à la résolution de l'ester résultant pour obtenir un alcool optiquement actif qui contient de manière enrichie soit le R-alcool ou le S-alcool.

2. Un procédé tel que revendiqué dans la revendication 1, caractérisé en ce que l'enzyme est une lipase du Pseudomonas genus.

3. Un procédé tel que revendiqué dans la revendication 1 ou 2, caractérisé en ce que Y de la formule (1) est un groupement alkyle ayant de 1 à 3 atomes de carbones.

4. Un procédé tel que revendiqué dans la revendication 1 ou 2, caractérisé en ce que Y de la formule (1) est $CH_3$ ou $C_2H_5$.

5. Un procédé tel que revendiqué dans la revendication 2, caractérisé en ce que Y de la formule (1) est $CF_3$.